# EUROPEAN PATENT APPLICATION

(11) **EP 1 889 612 A2**
(43) Date of publication of application: **20.02.2008**
(21) Application number: 06117729.1
(22) Date of filing: 24.07.2006
(51) Int. Cl.: A61K 31/00, A61K 31/198, A61P 13/10

(54) **Method for controlling micturition**

(71) Applicant: Bruinstroop, Jan Kees Piet, 2111 AA Aerdenhout (NL)
(72) Inventor: Bruinstroop, Jan Kees Piet, 2111 AA Aerdenhout (NL)
(74) Representative: van Westenbrugge, Andries

(57) **Abstract**

The present invention concerns the stimulation of neural tissue, in particular brain tissue for the control of micturition. By activation of the pontine micturition centre (PMC) to induce micturition and deactivation of the pontine micturition centre to inhibit micturition, patients suffering from overactive bladder (OAB) and urge incontinence regain control of the disposal of urine. This treatment will greatly improve these patients' quality of life and helps them to restore their self-confidence.

## Description

### FIELD OF THE INVENTION

The present invention concerns the stimulation and inhibition of neural tissue, in particular brain tissue for the control of micturition.

### BACKGROUND OF THE INVENTION

Micturition or emptying the bladder is necessary to dispose of urine, a fluid that contains waste substances. The brain and brainstem play a crucial role in the control of micturition and this importance is also reflected in one of the greatest problems of the ageing human body in modem society: overactive bladder (OAB) and urge-incontinence.

OAB affects more than 16.6% of all men and women over 40 years of age. OAB is defined by three main symptoms: frequency, urgency, and urge incontinence. Patients need to urinate so frequently that work, socializing and relationships are seriously hampered. Patients may not get enough sleep, as they wake up several times a night to empty their bladders. Urgency occurs suddenly, immediately followed by contraction of the detrusor muscle, leaving OAB patients with little time to reach the bathroom. These contractions can also cause urge incontinence, when the bladder suddenly voids. Fear that this will happen in public leaves many OAB sufferers housebound. In the USA alone the costs of OAB in the year 2000 amounted $12.6 billion and of urge-incontinence $19.5 billion, together in excess of $32 billion (Hu et al., (2004), Urology 63: 461-465). This huge sum is not only the result of the massive amount of diapers and incontinence materials that has to be acquired, but also because incontinence often is a decisive factor in that many elderly patients will require admittance into a nursing home to receive proper care instead of being able to live at home.

The greatest problem, however, is not the monetary problem, but the emotional suffering of urge incontinence patients due to feelings of shame, loneliness and total desperation. It is stressed that at the heart of the present invention lies the common notion that incontinence is something to be ashamed of. As a consequence patients do not openly speak out about it and do not nominate it as a problem. Patients that do seek help obviously first turn to an urologist. Urologists, however, have proven to be unable to provide effective solutions to incontinence problems, basically because, as will be explained further below, incontinence is not a urological but rather a neurological problem. Thus, historically there is a mismatch between the problem of incontinence, which has a neurological cause, and the urological direction in which a solution to the problem is sought. However, on the other hand neurologists never get to see patients having only incontinence problems. Effectively tackling the problem of incontinence requires the skills of uro-neurologists, which is yet virgin territory and a frontier of knowledge that yet has to be conquered.

Micturition is a combination of contraction of the detrusor muscle of the bladder and relaxation of the bladder sphincter. The bladder is innervated by parasympathetic preganglionic motoneurons in the sacral spinal cord. These motoneurons belong to the autonomic nervous system, hence these neurons cannot be excited voluntarily, and thus the bladder cannot be contracted at one's own free will. This is not so for the external sphincter of the bladder which consists of striated muscles and is innervated by somatic motoneurons, also located in the sacral spinal cord. The external striated bladder sphincter is a peculiar muscle, because on the one hand it can be contracted voluntarily, but on the other hand it must be precisely coordinated with bladder activity, which is controlled by the autonomic motor system. Also sympathetic preganglionic motoneurons innervate the bladder. Sympathetic fibers have inhibitory effects on the detrusor muscle and excitatory effects on the smooth musculature of urethra and base of the bladder. The sympathetic bladder innervation is thought to decrease bladder pressure during bladder filling in order to prevent reflexive contractions (see for review G. Holstege, Journal of Comparative Neurology, 493:15-20 (2005), deGroat et al (1999) Basic neurophysiology and neuropharmacology. In: P. Abrams, S. Khoury, A. Wein (Eds.) Incontinence. Plymbridge Distributors Ltd, Plymouth, pp. 107-154).

Thus, for micturition to occur, a system is needed that not only controls the somatic bladder sphincter motoneurons, but also the parasympathetic bladder motoneurons.

The pontine micturition center (PMC) in the brainstem is the only cell group that specifically controls the motoneurons of the bladder as well as the motoneurons of the bladder sphincter This cell group is located in the dorsolateral pontine tegmentum, just ventral to the mesencephalic trigeminal tract and locus coeruleus, (Holstege et al. (1986) J. Comp. Neurol., 250: 449-461), and is also called M-region or Barrington's nucleus, after Barrington's description in 1925 of a micturition control region in this area in the cat.

The PMC has a direct excitatory effect on bladder motoneurons, and, via gamma-aminobutyric acid (GABA)-ergic and glycinergic interneurons in the sacral cord, an inhibitory effect on the bladder sphincter motoneurons in the sacral cord. Via this pathway the PMC neurons not only can contract the detrusor muscle of the bladder, but also, at the same time, relax the bladder sphincter muscle, which, together, leads to micturition.

Since the brainstem plays such a crucial role in micturition control, it needs very precise and continuous information about the amount of urine in the bladder. For this reason, afferent A∂ fibers from mechanoreceptors in the bladder wall reach the spinal cord via the pelvic nerve to terminate in a distinct region just lateral to and in the lateral dorsal horn (Morgan et al., (1981), J. Comp. Neurol., 201: 415-440; DeGroat et al., (1982), J. Auton. Nerv. Syst., 523-5431982). This region not only contains a great number of dendrites of the parasympathetic bladder motoneurons, but also neurons that relay this bladder filling information to supraspinal levels, more specifically to the mesencephalic periaqueductal gray (PAG; Blok et al., (1995) J. Comp. Neurol., 359: 300-309; VanderHorst et al., (1996), J. Comp. Neurol. 376: 361-385; Mouton and Holstege, (2000), J Comp Neurol. 428: 389-410). This continuous stream of afferent information from the bladder to the PAG is not interrupted in people suffering from OAB or urge-incontinence, because their pelvic nerves, spinal cord and brainstem are intact. Therefore, it is unlikely that OAB patients have a problem with the stream of information from the bladder to the brain.

It has been shown (Holstege et al. (1986) vide supra) that there is a direct relationship between the PMC activation and micturition. Electrical stimulation of the PMC in cats always results in micturition, while micturition does not occur when the PMC is not artificially stimulated or, as is the case in normal situations, activated by other brain structures. The PMC does not receive direct projections from the sacral spinal cord, but is the target of very strong projections from the PAG (Blok and Holstege (1994), Neurosci. Lett., 166: 93-96; Blok et al, (1995) vide supra). Indeed, the PAG plays a crucial part in the micturition reflex. It is important that within the PAG, the central area that receives direct sacral cord projections differs from the more laterally located PAG area with cells that send projections to the PMC. Thus, the bladder information is first processed in the PAG before it is relayed to the PMC-projecting cells. In simple terms: the bladder sends a continuous stream of information about bladder filling to the PAG, where it is decided whether or not the amount of urine in the bladder is such that PMC projecting cells in the PAG will be activated.

This decision in the PAG is greatly influence by the great many descending influences of a large number of more rostrally located brain regions, mainly belonging to the limbic system or related to it. It means that the emotional environment in healthy patients determine whether or not micturition will take place.

At present common treatment for OAB includes medication that relaxes muscles and prevents bladder spasms, so-called bladder relaxants, such as oxybutynin chloride (Ditropan®) and tolterodine tartrate (Detrol®). These drugs mainly inhibit the parasympathetic innervation of organs, resulting not only in a decrease of bladder activity but also of all other parasympathetically innervated organs leading to dry mouth (less production of saliva), or blurred vision (less innervation of the sphincter of the pupil). Instead of or in combination with the bladder relaxants, a tricyclic antidepressant that relaxes bladder muscles and tightens urethral muscles is used, in particular imipramine hydrochloride (Tofranil®). Also pelvic floor muscle electrical stimulation (PFES), is applied to control the bladder and sphincter muscles.

For people with urge incontinence that do not respond to medical treatment a surgical technique called sacral nerve stimulation has been developed. This involves the implantation of a device that electrically stimulates the nerves that control voiding function. The reason why this stimulation would work in urge-incontinent patients is not known.

### SUMMARY OF THE INVENTION

It is considered that OAB is a brain disease caused by lesions in brain areas that influence a specific group of neurons in the dorsolateral pontine tegmentum in the brainstem, the so-called pontine micturition center (PMC).

At the basis of the present invention lies the inventive insight that because of the processing of information in the PAG before it is relayed to the PMC a great many other brain structures influence the sacral cord-PAG-PMC micturition system. According to the present invention, it is the inadequate communication between the forebrain and the PAG that accounts for the "lack of warning" that OAB patients report. The resolution of this simple conflict between cognitive-social-emotional intent and basic bodily needs lies at the core of the problem and it is in simple circuitry such as exists at the level of the PAG-PMC that the final behavioral pattern is resolved. Humans place remarkable social importance on this outcome, as judged by the acute embarrassment associated with wetting one's pants when excited or frightened, from childhood onward. This social ostracism is also at the core of the discomfiture of OAB patients who lose the ability to maintain this control.

The decision in the PAG whether or not to start micturition by exciting the PMC neurons, is not only influenced by the amount of bladder filling, but also by the many other brain regions that have direct access to the PAG. Examples are the lateral, caudal and anterior parts of the hypothalamus, the preoptic region (medial and lateral), the central nucleus of the amygdala, the bed nucleus of the stria terminalis, and large portions of the prefrontal cortex. All these brain regions take part in or are related to the limbic system, and continuously verify whether the safety of the environment is such that micturition can take place. If safety is not guaranteed, these limbic structures will prevent the PAG from exciting the PMC neurons, even when other parts of the PAG receive a strong message from the sacral cord that the bladder is full and needs to be emptied as soon as possible.

Thus, the decision to initiate micturition is made in the PAG, but not only based on afferent information from the sacral cord but also from brain structures belonging to or associated with the limbic system and prefrontal cortex. In more simple terms, although the PAG is continuously informed about the amount of bladder filling, its decision to initiate micturition is strongly influenced by the limbic system and prefrontal cortex that continuously evaluates the safety of the environment, which, in humans in particular, not only relates to bodily safety, but also to social safety. In healthy people these signals from the limbic system and prefrontal cortex are very powerful, but in OAB patients, because of reasons that may differ from patient to patient, these signals are weak. In such patients the strength of the "safety signals" might be so low that the spinal-brainstem-spinal micturition pathway acts more or less independent of the social environment, with increased feelings of urge and later on urge-incontinence as a consequence.

This concept also explains why reducing bladder activity by antimuscarinic drugs, at present the only drugs available to treat OAB and urge-incontinence, do not alleviate the symptoms in the long run. The same is true for interfering with the stream of afferent bladder information by means of stimulation of the pelvic nerve, because the afferent information from the bladder is not abnormal and the bladder is not overactive by itself, but is caused by lack of inhibition of the PAG-PMC pathway. Many elderly people suffer from various diverse and often small lesions in the various limbic structures or their descending limbic pathways to the PAG, preventing the usual inhibitory "safety" signals from reaching the PAG properly. Thus, the problem in OAB or urge-incontinence is due to the lack of inhibitory afferents to the PAG or PMC.

It is the object of the present invention to provide subjects with the active control over their disposing of urine. Hence, in other words, it is the object of the present invention to provide subjects with methods and/or means to by-pass the neurological pathways that occur in case of OAB and/or urge incontinence and allow patients to control micturition, in particular to allow patients to activate micturition and/or to allow patients to inhibit micturition at a moment chosen at free will by the patient or subject. In a sense it is the object of the present invention to provide a method and/or means to control the neurological pathway of micturition that occurs in subjects not suffering from OAB and/or urge incontinence.

The invention thus relates to a method for the control of micturition said method comprising a step selected from the group consisting of activation of the pontine micturition centre (PMC) to induce micturition and deactivation of the pontine micturition centre to inhibit micturition. In one embodiment activation and deactivation of PMC includes activation and deactivation of the neuronal axon(s) from PMC to the spinal cord.

The present invention has an enormous economical advantage in view of the reduction of costs it will bring about that are currently made in connection with OAB and urge incontinence. More importantly however, the present invention helps patients suffering from OAB and/or urge incontinence to restore their self-confidence and to improve their quality of life.

### DETAILED DESCRIPTION OF THE INVENTION

In scheme 1 a schematic overview of micturition control systems is given. Several sites have been indicated that serve as a target for the control of micturition. These target sites are the pontine micturition centre D and the neuronal axon extending from the PMC to the spinal cord, the periaqueductal gray, in particular the lateral periaqueductal gray (B), and the neuronal axon extending from PAG to PMC.

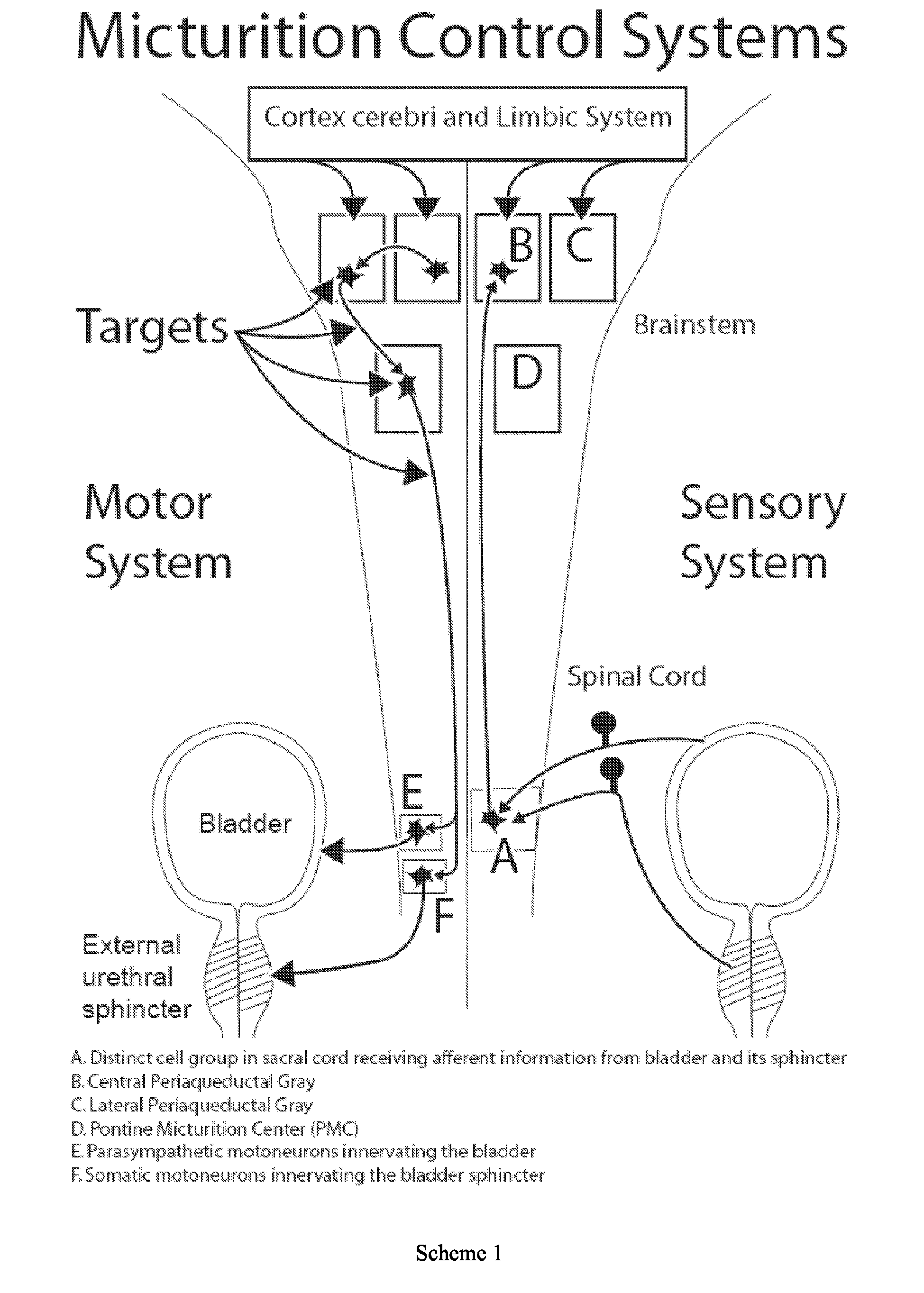

In a first embodiment the present invention uses electrical stimulation to control micturition. The electrical stimulation can be provided by an implantable electrode such as for instance described in US 6,227,203. The electrode is preferably implanted in the brain such that it is able to deliver electrical stimulation to the pontine micturition centre (PMC). In one embodiment the electrode can be operated by means outside or is reachable from the outside of the body of the subject having the electrode implanted. Signal strength, e.g. determined in terms of pulse width, frequency and amplitude can be adjusted such that micturition is induced. Alternatively by varying pulse width, frequency and amplitude micturition can be inhibited, for example by varying continuous and discontinuous signaling with varying signal strength. This embodiment can be operated by the patient at free will at a voluntarily chosen moment.

In one embodiment the control micturition by activation of PMC or deactivation PMC is brought about by activation of periaqueductal gray (PAG) and/or deactivation of PAG. In one embodiment activation and deactivation of PAG includes activation and deactivation of the neuronal axon(s) from PAG to PMC. In one embodiment the lateral part of PAG is activated and/or deactivated. In one embodiment electrical stimulation is delivered to PAG, for example by means of an implantable electrode such as for instance described in US 6,227,203, thereby providing the signal from the limbic system that it is not safe for micturition. In one embodiment such a signal is delivered to inhibit the naturally functioning signaling pathway from PAG to PMC which results in micturition. In a particular embodiment electrical stimulation is continuously delivered to PAG, thereby controlling micturition. In a preferred embodiment a patient can disrupt such a continuous signal thereby removing the inhibiting signal resulting in micturition. In a further embodiment the patient can deliver a signal by electrical stimulation means to the PMC after the inhibiting signal to PAG has been disrupted and preferably while this inhibiting signal is disrupted. In yet a further embodiment electrical stimulation is continuously delivered to PAG and the patient is able to deliver or in fact delivers a signal to PMC which so to speak overrides the inhibiting signal thereby inducing micturition. Thus in one embodiment the control of micturition by activation of PMC or deactivation PMC is brought about by a combination of electrical stimulation, for example by means of an implantable electrode such as for instance described in US 6,227,203, of PMC and PAG.

In an alternative embodiment the control of micturition by activation of PMC or deactivation of PMC is brought about by a stimulating or inhibiting compound that is delivered to a predetermined site in the brain by means of an implantable pump and a catheter which is connected to the pump and having a discharge end at the predetermined site in the brain. A suitable system is described also in US 6,227,203. In one embodiment the predetermined site in the brain for delivering compound for stimulation and inhibition is the PMC. Suitable stimulating compounds are glutamate receptor agonists such as in particular glutamate, or gamma-amino butyric acid (GABA)-antagonists. Suitable inhibiting compounds are gamma-amino butyric acid (GABA) receptor agonists such as in particular gamma-amino butyric acid (GABA) and glycine receptor agonists such as in particular glycine.

Also the PAG may be considered as the predetermined site in the brain for delivering a stimulating or inhibiting compound, optionally in combination with delivering a stimulating or inhibiting compound to PMC. For example a stimulating compound can be delivered to PAG thereby providing the signal from the limbic system that it is not safe for micturition. In a further embodiment a patient can disrupt the delivery of such a stimulating compound to PAG thereby removing the inhibiting signal resulting in micturition. In an alternative embodiment an inhibiting compound can be delivered to PAG that counteracts the effect of the stimulating compound. In another embodiment a stimulating compound can be delivered to PMC to induce micturition while a constant level of stimulating compound that imitates the signal from the limbic system that it is not safe for micturition is maintained in PAG. In this embodiment the stimulating compound delivered to PMC so to speak overrides the inhibiting signal from PAG.

In a further embodiment the control of micturition by activation of PMC or deactivation PMC is brought about by a combination of electrical stimulation and stimulating and/or inhibiting compounds. Thus embodiments of the method of the invention are a combination of electrical stimulation by means of an implanted electrode of PAG and delivering a stimulating compound as defined above to PMC or electrical stimulation of PAG and delivering an inhibiting compound as defined above to PMC or delivering a stimulating compound as defined above to PAG and electrical stimulation by means of an implanted electrode of PMC or delivering an inhibiting compound as defined above to PAG and electrical stimulation by means of an implanted electrode of PMC. For example a continuous inhibiting electrical signal is delivered to PAG. At a moment of choice the patient can deliver a stimulating compound to PMC thereby inducing micturition. Alternatively a stimulating electrical signal can be delivered to PMC while a constant level of stimulating compound that imitates the signal from the limbic system that it is not safe for micturition is maintained in PAG.

Thus in one aspect the invention relates to the use of a compound for the preparation of a medicament for the control of micturition, said compound being selected from the group consisting of a compound that is capable of stimulating the pontine micturition centre (PMC), a compound that is capable of stimulating periaqueductal gray (PAG), a compound that is capable of inhibiting the PMC and a compound that is capable of inhibiting PAG.

In one aspect the invention relates to the use of glutamate receptor agonists such as in particular glutamate, or gamma-amino butyric acid (GABA)-antagonists for the preparation of a composition for the control of micturition. Further the invention concerns the use of gamma-amino butyric acid (GABA) receptor agonists such as in particular gamma-amino butyric acid (GABA) and glycine receptor agonists such as in particular glycine for the preparation of a composition for the control of micturition. Preferably said compositions are suitable for delivery to a site in the brain.

In one embodiment the control of micturition according to the present invention is for the treatment of overactive bladder (OAB) or more in particular for the treatment of urge incontinence.

## Claims

1. Use of a compound for the preparation of a medicament for the control of micturition, said compound being selected from the group consisting of a compound that is capable of stimulating the pontine micturition centre (PMC), a compound that is capable of stimulating periaqueductal gray (PAG), a compound that is capable of inhibiting the PMC and a compound that is capable of inhibiting PAG.

2. Use according to claim 1 wherein the compound that is capable of stimulating the PMC and or PAG is a glutamate receptor agonist.

3. Use according to claim 2 wherein the glutamate receptor agonist is glutamate.

4. Use according to claim 1 wherein the compound that is capable of stimulating the PMC and or PAG is a gamma-amino butyric acid (GABA) receptor antagonist.

5. Use according to claim 1 wherein the compound that is capable of inhibiting the PMC and or PAG is a gamma-amino butyric acid (GABA) receptor agonist.

6. Use according to claim 5 wherein the gamma-amino butyric acid (GABA) receptor agonist is gamma-amino butyric acid (GABA).

7. Use according to claim 1 wherein the compound that is capable of inhibiting the PMC and or PAG is a glycine receptor agonist.

8. Use according to claim 7 wherein the glycine receptor agonist is glycine.

9. Use according to any of the preceding claims wherein the composition is delivered to the PMC and/or PAG by means of an infusion device.

10. *Use according to any of the preceding claims wherein the composition is for the treatment of overactive bladder (OAB) or urge incontinence.*
